# FASCICULE DE BREVET EUROPEEN

(11) **EP 1 913 988 B1**
(45) Date de publication et mention de la délivrance du brevet: **23.06.2010**
(21) Numéro de dépôt: 07291223.1
(22) Date de dépôt: 08.10.2007
(51) Int. Cl.: B01D 15/18, C07C 7/12, G01N 30/44

(54) **Procédé et dispositif de séparation en lit mobile simulé à nombre de vannes de grand diametre reduit**
Verfahren und Vorrichtung zur Abscheidung in einem simulierten Fließbett mit einer reduzierten Anzahl von Schiebern großen Durchmessers
Separation method and device in a simulated entrained bed with a reduced number of large diameter valves

(30) Priorité: 16.10.2006 FR 0609192
(43) Date de publication de la demande: 23.04.2008
(73) Titulaire: IFP, 92852 Rueil-Malmaison Cédex (FR)
(72) Inventeur: Hotier, Gérard, 92500 Rueil Malmaison (FR); Leflaive, Philibert, 69780 Mions (FR); Louret, Sylvain, 69007 Lyon (FR); Augier, Frédéric, 69360 Saint Symphorien D'Ozon (FR)

(56) Documents cités:
- EP-A- 1 325 772
- EP-A1- 0 923 970
- FR-A1- 2 782 657
- US-A1- 4 434 051

## Description

### Domaine de l'invention:

L'invention se rapporte au domaine des séparations de produits naturels ou chimiques, que l'on peut difficilement séparer par distillation. On utilise alors une famille de procédés, et de dispositifs associés, connus sous le nom de procédés, ou dispositifs de séparation « chromatographique », ou « en lit mobile simulé », ou « en contre-courant simulé », ou « en co-courant simulé », que nous désignerons ci-après par l'appellation « SMB ».

Les domaines concernés sont notamment, et de façon non exclusive :
- la séparation entre d'une part les paraffines normales et d'autre part les paraffines ramifiées, naphtènes, et aromatiques,
- la séparation oléfines / paraffines,
- la séparation du paraxylene des autres isomères en C8 aromatiques,
- la séparation du métaxylene des autres isomères en C8 aromatiques,
- la séparation de l'éthylbenzene des autres isomères en C8 aromatiques.

Hors raffinerie et complexe pétrochimique il existe de nombreuses autres applications parmi lesquelles on peut citer la séparation glucose / fructose, la séparation des isomères de position du crésol, des isomères optiques etc...

### Art antérieur :

La séparation chromatographique SMB est bien connue dans l'état de la technique. En règle générale, un lit mobile simulé comporte au moins trois zones chromatographiques, avantageusement quatre ou cinq ou six zones, chacune de ces zones étant constituée par au moins un lit ou une portion de colonne et comprise entre deux points successifs d'alimentation ou soutirage. Typiquement, on alimente au moins une charge F à fractionner et un désorbant D (parfois appelé éluant) et l'on soutire au moins un raffinat R et un extrait E. Parfois, on alimente également un reflux RE riche en extrait. On peut également utiliser non pas un raffinat R, mais deux raffinats R1 et R2. Il y a donc généralement 4, 5, ou 6 fluides de procédés, alimentés ou soutirés de façon séquentielle. Les points d'alimentation et de soutirage sont modifiés au cours du temps, décalés dans le sens de l'écoulement et typiquement vers le bas d'un lit, et ce de façon synchrone.

Plusieurs variantes avantageuses permettent d'améliorer le fonctionnement de ce type d'unité en faisant appel à des permutations asynchrones. De façon simplifiée, ces permutations asynchrones servent à compenser le(s) volume(s) mort de(s) la pompe(s) de recirculation, tel qu'indiqué dans le brevet US 5,578,215, à travailler avec un débit de recyclage constant sur la pompe de recirculation de manière à eliminer les à-coup de débit et de pression, tel qu'indiqué dans le brevet US 5,762,806, ou enfin à opérer avec au moins deux zones chromatographiques dont chacune est équivalente à un nombre non entier de lits d'adsorbant. Cette dernière variante, tel qu'indiquée dans les brevets US 6,136,198, US 6,375,839, US 6,712,973, et US 6,413,419 est connue sous le nom de Varicol. Naturellement, ces trois variantes peuvent être combinées.

Il est à noter qu'une vanne rotative multivoie mettant en communication d'une part les fluides entrants et sortants et d'autre part les lits disposés dans la ou les colonnes d'adsorption ne permet qu'une permutation de type synchrone. Pour des permutations asynchrones, une pluralité de vannes tout ou rien est indispensable. Cet aspect technologique est exposé plus loin.

L'état de la technique décrit de façon approfondie différents dispositifs et procédés permettant d'effectuer la séparation de charges en lit mobile simulé. On peut citer notamment les brevets US 2,985,589, US 3,214,247, US 3,268,605, US 3,592,612, US 4,614,204, US 4,378,292, US 5,200,075, US 5,316,821. Ces brevets décrivent également en détail le fonctionnement d'un SMB.

Les dispositifs SMB comportent typiquement au moins une colonne (et souvent deux), des lits d'adsorbant Aᵢ disposés dans cette colonne, séparés par des plateaux Pᵢ à chambre(s) Ci de distribution et/ou d'extraction de fluides dans ou à partir des différents lits d'adsorbant, et des moyens commandés de distribution et d'extraction séquentiels de fluides.

Chacun des plateaux Pᵢ comprend typiquement une pluralité de panneaux distributeurs-mélangeurs-extracteurs ou « DME » alimentés par des lignes ou « araignées de distribution/extraction ». Les plateaux peuvent être de tout type et de toute géométrie, notamment à panneaux formant des secteurs adjacents de la section de la colonne, par exemple des panneaux à secteurs angulaires tels que présentés dans le brevet US 6,537,451 figure 8, qui sont à alimentation (araignée) symétrique, ou des secteurs parallèles tels que découpés dans une circonférence, ainsi qu'indiqué dans la demande publiée de brevet US 03/0,127,394, qui sont à alimentation disymétrique. De façon préférée, la colonne de séparation comprend des plateaux à DME de type à secteurs parallèles et alimentations disymétriques. De façon également préférée, l'adsorbant est installé en chargement dense. Ceci permet d'utiliser une plus grande quantité d'adsorbant dans une colonne donnée, et d'accroître la pureté du produit recherché et/ou le débit de charge du SMB.

La distribution sur chacun des lits requiert une collecte du flux provenant du lit précédent (fluide principal circulant selon l'axe principal de la colonne), la possibilité d'y injecter un fluide annexe ou fluide secondaire tout en mélangeant le mieux possible ces deux fluides, ou encore la possibilité de prélever une partie du fluide collecté, de l'extraire pour l'envoyer vers l'extérieur du dispositif et aussi de redistribuer un fluide sur le lit suivant.

Pour ce faire, on peut utiliser dans un plateau Pᵢ des chambres Ci,k de distribution (injection / extraction) qui peuvent être séparées ou communes avec des chambres de mélange. On connait des plateaux Pᵢ à une ou plusieurs chambres, soit alimentées (ou soutirées) séparément par des fluides différents à un instant donné, soit alimentées (ou soutirées) simultanément et en parallèle par le même fluide à un instant donné. Dans le premier cas, on dit que le plateau est à plusieurs réseaux de distribution, alors qu'il est à réseau de distribution unique dans le second cas. L'invention se rapporte exclusivement à un dispositif comprenant des plateaux à réseau de distribution unique.

De façon générale, on peut soit faire transiter l'intégralité du fluide ou flux principal dans la colonne selon un schéma décrit dans le brevet US 2,985,589, soit faire ressortir une grande partie ou la totalité de ce flux vers l'extérieur selon un procédé décrit dans le brevet US 5,200,075.

Un problème générique de l'ensemble des dispositifs SMB est de minimiser la pollution générée par le liquide se trouvant dans les différentes zones et volumes des circuits d'alimentation et de soutirage de fluides des plateaux, lors des modifications des points d'alimentation et de soutirage au cours du fonctionnement du SMB. En effet, lorsque, au cours de la séquence de fonctionnement, une ligne, chambre, ou zone d'alimentation d'un plateau Pᵢ n'est plus balayée par un fluide de procédé, elle devient une zone morte dans lequel le liquide stagne, et n'est remis en mouvement que lorsqu'un autre fluide de procédé y circule à nouveau. Comme, de par le fonctionnement du SMB, il s'agit alors d'un fluide de procédé qui est différent, le liquide de la zone morte est nécessairement déplacé par un liquide de composition notablement différente. Le mélange, ou la circulation à bref intervalle de temps de fluides de compositions notablement différentes introduit donc une perturbation par rapport au fonctionnement idéal, pour lequel les discontinuités de composition sont à proscrire.

Un autre problème peut résider dans les éventuelles recirculations entre différentes zones d'un même plateau, ce qui induit alors également une perturbation par rapport au fonctionnement idéal.

Pour résoudre ces problèmes liés aux recirculations et aux zones mortes, différentes techniques sont déjà connues de l'art antérieur :
a) Il a déjà été proposé de réaliser un balayage (on utilise souvent également le mot anglais « flush ») des lignes et zones mortes par notamment du désorbant ou du produit recherché, relativement pur. Cette technique permet effectivement d'éviter la pollution du produit désiré lors de son extraction. Toutefois, comme le liquide de balayage a typiquement une composition très différente du liquide qu'il déplace, cela introduit des discontinuités de composition préjudiciables au fonctionnement idéal. Cette première variante de balayage réalise typiquement des « balayages de courte durée à gradient de concentration élevé ». Ces balayages sont de courte durée pour limiter les effets des discontinuités de composition.
b) Une autre solution consiste, comme il est décrit dans le brevet US 5,972,224, à faire transiter une majorité du flux principal vers l'intérieur de la colonne et une minorité de ce flux vers l'extérieur, typiquement de 2 % à 20 % du flux, par des lignes de dérivation externes entre plateaux voisins. Ce balayage est typiquement réalisé pendant la majorité du temps ou en continu, de telles sorte que les lignes et zones ne soient plus « mortes », mais balayées. Un tel système avec balayage via des lignes de dérivation est présenté à la figure 1 du brevet US 5,972,224 et repris de façon simplifiée à la figure 1 de la présente demande. Comme les lignes de dérivation sont prévues pour un débit faible, elles peuvent être en conséquence de petit diamètre, et comprendre une vanne de petit diamètre, ce qui réduit le coût du système.

Un premier avantage d'un tel système est que les circuits d'injection et de prélèvement des fluides secondaires sont balayés par du liquide ayant une composition très voisine du liquide déplacé puisque d'une part la dérivation provient du plateau voisin, et d'autre part qu'il y a balayage non pas ponctuel mais sensiblement continu. De plus, on détermine de préférence les débits dans les dérivations de façon à ce que la vitesse de transit dans chaque dérivation soit sensiblement la même que la vitesse d'avancement du gradient de concentration dans le flux principal du SMB. Ainsi, d'une part on réalise un balayage des différentes lignes et capacités par un fluide qui a une composition sensiblement identique à celle du liquide qui s'y trouve, et d'autre part l'on réintroduit le liquide circulant dans une dérivation en un point ou la composition du flux principal est sensiblement identique. Cette deuxième variante réalise donc des « balayages de longue durée à gradient de concentration faible ou nul ».

Un second avantage de ce système à balayages de longue durée (hors périodes d'injection ou de soutirage), est qu'il permet de supprimer les effets de possibles recirculations entre zones d'un même plateau, dues à de petites différences de pertes de charge.

Pour ce qui concerne le fonctionnement d'un SMB, les moyens commandés de distribution et d'extraction de fluides d'un SMB sont typiquement de l'un des deux grands types suivants de technologie :
- soit, pour chaque plateau, une pluralité de vannes commandées tout ou rien pour l'alimentation ou le soutirage des fluides, ces vannes étant typiquement situées au voisinage immédiat du plateau correspondant, et comprenant notamment, pour chaque plateau Pᵢ au moins 4 vannes commandées tout ou rien à 2 voies pour respectivement les alimentations des fluides F et D et les soutirages des fluides E et R.
- soit une vanne rotative multi-voies pour l'alimentation ou le soutirage des fluides sur l'ensemble des plateaux.

La première technologie utilise des vannes à 2 voies, ce qui permet une fabrication standard en série conduisant à une fiabilité accrue et à un coût unitaire relativement faible. La seconde technologie n'utilise qu'une vanne unique, mais cette vanne unique est multivoie (comporte plus de 2 voies), et nécessairement de construction spéciale, de grande dimension et de complexité élevée. De plus cette seconde technologie exclut la possibilité de permutations asynchrones, comme dans les dispositifs Varicol.

L'invention se rattache aux SMB utilisant des vannes conventionnelles à 2 voies, c'est-à-dire utilisant la première des deux technologies décrites ci-dessus. Elle concerne en particulier un dispositif perfectionné de séparation en lit mobile simulé comprenant une pluralité de vannes à 2 voies, à nombre de vannes commandées généralement un peu réduit, et surtout en nombre notablement réduit pour les vannes commandées de grand diamètre d'ouverture, par rapport à l'art antérieur. Elle est utilisable aussi bien pour un SMB à permutations synchrones que pour un SMB à permutations asynchrones, par exemple un Varicol.

### Description simplifiée de l'invention :

L'invention concerne un dispositif perfectionné de séparation en lit mobile simulé appartenant au grand type technologique des SMB utilisant une pluralité de vannes commandées (tout ou rien ou à ouverture progressive) à 2 voies, typiquement des vannes standard fabriquées en série à coût réduit pour un niveau de qualité (étanchéité / fiabilité) élevé requis.

L'un des buts essentiels de l'invention est de réduire l'inconvénient relatif de ce type de SMB qui est de requérir un nombre élevé de vannes commandées à 2 voies de grand diamètre, c'est-à-dire de diamètre d'ouverture compatible avec la circulation des fluides de procédé du SMB à leur débit nominal. L'invention permet typiquement de réduire notablement le nombre de vannes commandées de grand diamètre d'ouverture, tout en conservant l'avantage de pouvoir mettre en oeuvre un balayage efficace des zones mortes du type «de longue durée à gradient de concentration faible ou nul ».

Un autre but de l'invention est de présenter un dispositif qui nécessite un nombre de vannes à 2 voies de grand diamètre (d'ouverture) réduit sans que ces vannes aient une fréquence d'ouverture fermeture / augmentée par rapport à la solution de l'art antérieur, ce qui compte tenu du nombre réduit de vannes de grand diamètre limite les risques statistiques de dysfonctionnement et accroît donc la fiabilité du système.

Enfin, une variante préférée du dispositif permet de réduire encore le nombre de vannes de grand diamètre qui permettent la circulation des principaux fluides du SMB à leur débit nominal.

Le dispositif selon l'invention peut être mis en oeuvre sur des installations neuves mais est également compatible avec diverses installations existantes sur lesquelles il peut être installé en réalisant des modifications limitées. Il est aussi compatible avec divers types et géométries de plateaux Pᵢ, par exemple des plateaux à panneaux à secteurs angulaires ou bien à secteurs parallèles, dans la mesure ou ces plateaux (ou la majorité d'entre eux) sont du type à réseau de distribution unique, pour l'alimentation ou le soutirage séquentiel d'un fluide de procédé du SMB.

Il a donc été trouvé un moyen permettant de réduire sensiblement le nombre des vannes commandées principales de grand diamètre, correspondant aux entrées sorties séquentielles des fluides de procédé du SMB à leur débit nominal : Dans l'art antérieur, il y a pour chaque plateau au moins un jeu de 4 vannes principales de réseau pour les alimentations / soutirages de F, D, R, E. Ce nombre est encore augmenté si l'on a plus de 4 fluides de procédé pour le SMB, par exemple si l'on a 2 raffinats R1, R2 et/ou si l'on utilise un reflux RE riche en produit recherché, typiquement en extrait. On a alors autant de vannes commandées de grand diamètre par plateau que de fluides de procédé du SMB, soit le plus souvent entre 4 et 6, bornes comprises.

Les lignes de dérivation, de petit diamètre, ne sont dans l'art antérieur que des lignes annexes qui ne sont pas empruntées par les fluides F, D, R, E (E1), (E2) (RE) à leur débit nominal d'alimentation ou de soutirage, mais par un débit notablement plus faible, typiquement moins de 20% du débit circulant dans la colonne, souvent entre 2% et 10% de ce débit. Elles comprennent donc typiquement une vanne commandée à ouverture progressive (pour contrôle du débit de balayage) de petit diamètre d'ouverture (ou diamètre équivalent de même section de passage).

Selon l'invention, on regroupe la colonne, ou une partie principale (plus de 50% de la hauteur de la colonne au moins) de cette colonne en tronçons Sₖ superposés, chaque tronçon Sₖ comportant 2 lits d'adsorbants successifs Aᵢ, Aᵢ₊₁, et les 2 plateaux Pᵢ, Pᵢ₊₁ situés respectivement immédiatement en dessous ce ces lits, et comprenant également une ligne de dérivation principale Lₖ. Contrairement à l'art antérieur, la ligne de dérivation Lₖ est empruntée par les fluides du SMB à leur débit nominal (et non pas seulement par un petit débit de balayage), et l'on utilise un seul jeu de vannes principales de réseau (d'alimentation ou soutirage séquentiel) par tronçon de colonne (pour 2 plateaux et non par plateau comme dans l'art antérieur), ces vannes de grand diamètre étant raccordées à la ligne de dérivation Lₖ de façon à permettre la circulation de ces fluides via Lₖ.

Selon l'invention, on prévoit également des "vannes de plateau", soit une vanne de grand diamètre, respectivement Vᵢ ou Vᵢ₊₁ pour chacun des plateaux Pᵢ, Pᵢ₊₁ de Sₖ, ainsi que des moyens additionnels de limiter les petits débits de fluide de balayage circulant dans Lₖ.

Selon l'invention, on prévoit également une ligne de dérivation secondaire Mₖ reliant le secteur Sₖ au secteur adjacent immédiatement inférieur Sₖ₊₁. Ceci permet un excellent balayage de l'ensemble des plateaux du SMB et tend à améliorer la pureté du produit récupéré, typiquement de l'extrait.

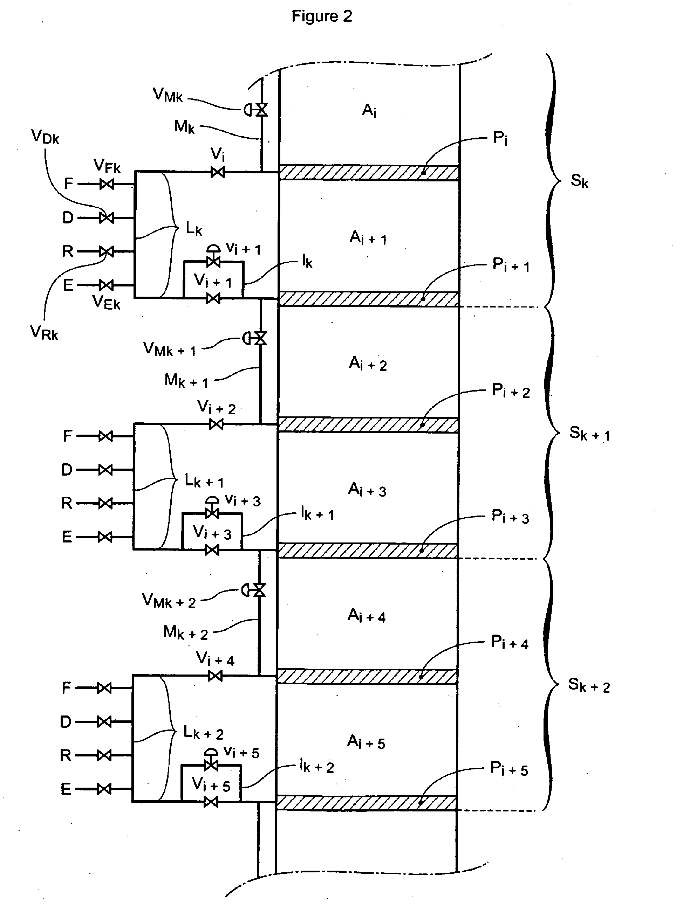

Comme il sera explicité ci-après, notamment pour la description de la figure 2, qui permet de comprendre plus aisément l'invention, le nombre total de vannes commandées de grand diamètre est réduit.

L'invention concerne également un procédé de séparation SMB utilisant le dispositif ainsi décrit, notamment pour la séparation d'un aromatique, en particulier du paraxylène ou du métaxylene à partir d'une charge d'hydrocarbures aromatiques à 8 atomes de carbone.

L'invention concerne également un procédé de séparation SMB utilisant le dispositif ainsi décrit, notamment pour la séparation d'un hydrocarbure normal-paraffinique, ou d'un hydrocarbure oléfinique à partir d'une coupe comprenant un tel hydrocarbure.

### Description détaillée de l'invention:

L'invention sera mieux comprise à la lecture de la description qui suit et en consultant les figures 1 (art antérieur) et les figures 2 et 3 (dispositif selon l'invention).

Pour réaliser l'un des buts précités, il est donc proposé selon l'invention un dispositif permettant de séparer au moins un composé à partir d'un mélange comprenant ce composé par adsorption en lit mobile simulé comportant :
au moins une colonne comprenant une pluralité de lits d'adsorbants Aᵢ séparés par des plateaux distributeurs/extracteurs Pᵢ pour l'alimentation et l'extraction séquentielles d'au moins deux fluides d'alimentation : une charge F et un désorbant D, et d'au moins deux fluides de soutirage : un raffinat R et un extrait E, Pᵢ étant disposé entre le lit Aᵢ et le lit immédiatement inférieur Aᵢ₊₁,
le dispositif comprenant également au moins un réseau F-Net de charge, un réseau D-Net de désorbant, un réseau R-Net de raffinat et un réseau E-Net d'extrait, chacun de ces réseaux étant relié à la colonne par une pluralité de lignes comprenant des vannes de sectionnement commandées à 2 voies de diamètre d'ouverture supérieur ou égal à une valeur α, appelées vannes de réseau, pour l'alimentation ou le soutirage séquentiels desdits fluides d'alimentation ou de soutirage,
dans lequel la colonne est divisée, sur la plus grande partie au moins de sa hauteur en une pluralité de tronçons superposés adjacents Sₖ, chaque tronçon Sₖ étant constitué essentiellement par 2 lits d'adsorbant successifs Aᵢ, Aᵢ₊₁ et par les 2 plateaux distributeurs/extracteurs Pᵢ, Pᵢ₊₁ qui sont disposés respectivement immédiatement en dessous de Aᵢ et de Aᵢ₊₁,
chacun des plateaux distributeurs/extracteurs Pᵢ, Pᵢ₊₁ de chacun des secteurs Sₖ est à réseau unique commun pour l'alimentation et le soutirage séquentiels de F, D, R, E,
les plateaux Pᵢ, Pᵢ₊₁ de chaque tronçon Sₖ sont reliés entre eux par une ligne de dérivation externe principale Lₖ connectée à chacun des plateaux Pᵢ, Pᵢ₊₁ de Sₖ par un embout comprenant une vanne de sectionnement commandée à 2 voies unique propre à ce plateau Pᵢ ou P_{i+1,} appelée vanne de plateau Vᵢ, ou Vᵢ₊₁, de diamètre d'ouverture supérieur ou égal à la valeur α pour l'alimentation ou le soutirage séquentiels desdits fluides d'alimentation ou de soutirage dans ou à partir de Pᵢ,
chacune des dites lignes de dérivation Lₖ comprend au moins un moyen commandé de limitation du débit circulant dans Lₖ, qui est soit installé sur la ligne Lₖ soit en dérivation autour d'une vanne de plateau Vᵢ ou Vᵢ₊₁ d'un plateau de Sₖ,
dans lequel la ligne de dérivation Lₖ de chacun des tronçons Sₖ est reliée à chacun des réseaux F-Net, D-Net, R-Net, E-Net via une ligne unique de diamètre interne supérieur ou égal à α comprenant une vanne de réseau unique, respectivement V_{Fk}, V_{Dk}, V_{Rk}, V_{Ek}, qui est de diamètre d'ouverture supérieur ou égal à α, pour l'alimentation ou le soutirage séquentiel du fluide correspondant F, D, R ou E vers ou à partir du tronçon Sₖ considéré,
le dispositif comprenant également une pluralité de lignes de dérivation externes secondaires Mₖ, chacune des lignes Mₖ reliant les 2 secteurs adjacents Sₖ₋₁ et Sₖ via 2 points de liaison,
le premier point de liaison étant disposé sur l'embout relié au plateau inférieur Pᵢ₋₁ du secteur supérieur Sₖ₋₁, entre Pᵢ₋₁ et la vanne de plateau Vᵢ₋₁,
le second point de liaison étant disposé sur l'embout relié au plateau supérieur Pᵢ du secteur inférieur Sₖ, entre Pᵢ et la vanne de plateau Vᵢ,
chacune des lignes de dérivation externes secondaires Mₖ comprenant une vanne commandée à 2 voies V_{Mk} de diamètre interne d'ouverture inférieur ou égal à une valeur β, avec β ≤ 0,6 α.

Typiquement α et β sont choisis de façon à vérifier l'inégalité suivante: 30 mm ≤ 1,7 x β ≤ α ≤ 600 mm. On voit que les vannes V_{Mk} de diamètre interne d'ouverture inférieur ou égal à β sont beaucoup plus petites et moins onéreuses que les vannes de diamètre interne d'ouverture supérieur ou égal à α.

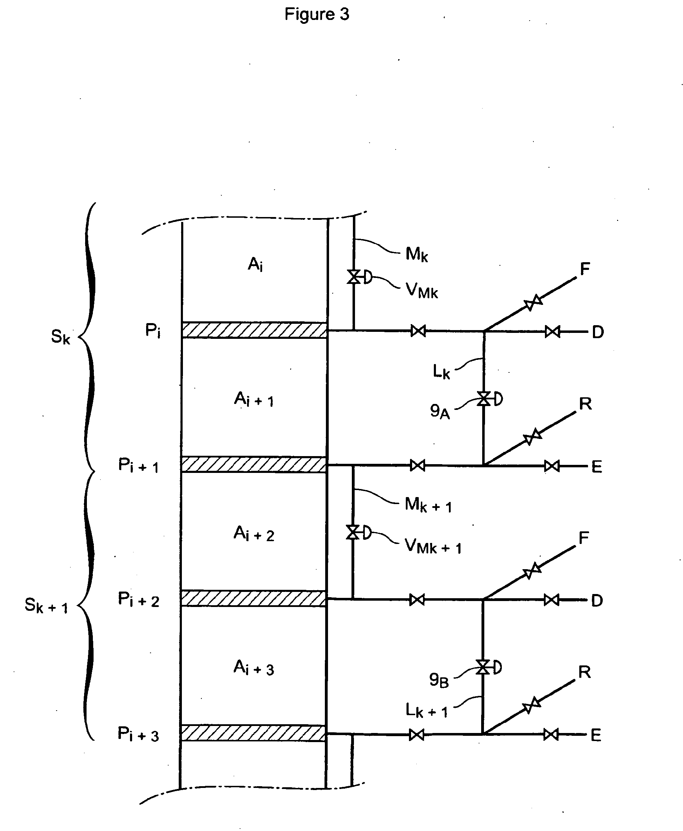

Contrairement au dispositif selon l'art antérieur, le dispositif selon l'invention permet d'utiliser la ligne de dérivation Lₖ pour la circulation des fluides F, D, R, E (et de préférence les autres fluides de procédé éventuels) alimentés au SMB et soutirés du SMB au niveau du tronçon Sₖ, via un jeu unique de vannes de réseau correspondantes, au lieu d'un jeu de vannes de réseau par plateau Pᵢ comme selon l'art antérieur. Ceci permet une réduction du nombre global de vannes commandées de grand diamètre, même en tenant compte de l'ajout de vannes supplémentaires, à savoir les vannes de plateau Vᵢ, comme il sera montré ci-après lors de la description des figures 2 et 3.

Les vannes commandées précitées: vannes de réseau et vannes de plateau Vᵢ sont typiquement des vannes de haute qualité (fiabilité, étanchéité, durée de vie) réalisant le fonctionnement séquentiel du SMB.

De façon plus générale, toutes les vannes commandées assurant le fonctionnement séquentiel du SMB : vannes de réseau, vannes Vᵢ de plateau, et aussi vannes des moyens commandés de limitation du débit circulant dans Lₖ doivent être considérées selon l'invention comme les vannes "principales" du SMB, reliées à la colonne et commandées par le système de commande du fonctionnement séquentiel du SMB (ordinateur, automate programmable ou autre système équivalent).

Certaines vannes principales du fonctionnement séquentiel du SMB ont été mentionnées précédemment comme étant uniques selon l'invention : Vᵢ pour chaque plateau Pᵢ ; jeu unique de vannes de réseau V_{Fk}, V_{Dk}, V_{Rk}, V_{Ek},..... pour chaque tronçon Sₖ. Ces vannes sont exclusivement celles permettant le fonctionnement séquentiel du SMB. On ne sortirait cependant pas du cadre de l'invention si l'on utilisait en sus d'autres vannes telles que des vannes secondaires d'isolement occasionnel, typiquement de qualité bien inférieure, commandées ou non, mais ne participant pas au fonctionnement séquentiel du SMB et permettant par exemple le démontage d'un équipement quelconque: pompe ou vanne principale utilisée pour le fonctionnement séquentiel etc....

Typiquement, la ligne de dérivation Lₖ, qui est utilisée pour le transit de tous les fluides F, D, R, E,...... à leur débit nominal n'est plus, dans le dispositif selon l'invention, une petite ligne annexe comme dans l'art antérieur, mais a généralement un diamètre intérieur au moins égal au plus grand diamètre d'ouverture des vannes de réseau reliées à Lₖ, de façon à pouvoir faire circuler les fluides F, D, R, E à leur débit nominal sans limitation de capacité. Le débit nominal d'un fluide de procédé est par définition le débit contrôlé de ce fluide, qui est utilisé au cours du fonctionnement séquentiel du SMB, pour la séparation voulue.

Du fait de l'utilisation de lignes de dérivation Lₖ aptes à véhiculer des débits nominaux relativement importants, on utilise avantageusement des moyens commandés de limitation du débit afin de réaliser également les circulations en dérivation dans Lₖ à faible débit (typiquement de 2 à 20% du débit circulant dans la colonne). Le terme "circulation en dérivation" signifie ici qu'une'fraction (faible) du débit circulant dans la colonne est soutirée d'un plateau et réintroduite sur un autre plateau du même tronçon Sₖ. Le terme moyens commandés s'applique typiquement à une vanne commandée, typiquement au moyen d'une chaîne de régulation, à partir de l'information fournie par un débitmètre.

On peut à cet effet utiliser une vanne de régulation de débit installée directement sur la ligne Lₖ, comme représenté sur la figure 3. Cette vanne est alors typiquement une vanne à ouverture progressive de grand diamètre, et non une vanne commandée tout ou rien (ayant seulement 2 positions possibles : pleine ouverture et fermeture).

Cependant, selon une variante préférée de l'invention, représentée à la figure 2, au moins une, ou de préférence chacune des lignes de dérivation Lₖ comprend un moyen commandé de limitation du débit circulant dans Lₖ, qui n'est pas installé directement sur Lₖ mais en dérivation autour d'une vanne de plateau de l'un plateau de Sₖ, par exemple autour de la vanne de plateau Vᵢ₊₁ du plateau inférieur Pᵢ₊₁. Ce moyen de limitation de débit, disposé sur une petite dérivation annexe *Iₖ* comprend généralement une vanne commandée *vᵢ₊₁* de plus petit diamètre d'ouverture que celui de Vᵢ₊₁, par exemple de diamètre d'ouverture au plus égal à 60%, ou à 50% de celui de Vᵢ₊₁, par exemple compris entre 10% et 50% du diamètre d'ouverture de Vᵢ₊₁.

La vanne *vᵢ₊₁,* est typiquement de diamètre d'ouverture inférieur ou égal à β et souvent inférieur ou égal à la moitié de α. Le dimensionnement de cette vanne de contrôle du débit de balayage est en effet avantageusement le même que celui de la vanne V_{Mk} disposée sur Mₖ. Dans les deux cas, il s'agit d'assurer la régulation d'un débit limité de balayage. Ainsi, de la même façon, chacune des lignes de dérivation secondaires Mₖ comprend typiquement au moins un moyen commandé de limitation du débit circulant dans Mₖ, ce moyen comprenant la vanne V_{Mk}.

Lorsqu'on veut effectuer un balayage interne en dérivation via Lₖ et limiter ce débit interne (circulant typiquement du plateau supérieur Pᵢ de Sₖ vers le plateau inférieur Pᵢ₊₁ de Sₖ), on laisse la vanne de plateau Vᵢ₊₁ fermée, l'on ouvre la petite vanne *vᵢ₊₁* en dérivation autour de Vᵢ₊₁, cette vanne *vᵢ₊₁* opèrant en contrôle de débit, et l'on ouvre Vᵢ, permettant ainsi la circulation d'un débit de balayage limité provenant de Pᵢ et recyclé dans Pᵢ₊₁ via Lₖ et Iₖ (voir figure 2).

Ainsi, l'utilisation d'une petite dérivation annexe *Iₖ* permet d'utiliser une vanne de plus petit diamètre d'ouverture que si le moyen de limitation de débit est installé sur la ligne de dérivation principale Lₖ, qui est de relativement plus grand diamètre du fait que Lₖ doit permettre la circulation des fluides F, D, R, E,... à leur débit nominal.

Selon l'invention, l'embout comprenant Vᵢ₊₁ doit être interprété comme ne comprenant pas la petite dérivation secondaire *Iₖ* autour de Vᵢ₊₁, ni la petite vanne *vᵢ₊₁* disposée sur *Iₖ.* Cet embout comprend donc bien une vanne unique Vᵢ₊₁ permettant la circulation des fluides principaux F, D, R, E,......

Typiquement, la ligne de dérivation Lₖ a un diamètre intérieur au moins égal au plus grand diamètre d'ouverture des vannes de réseau reliées à Lₖ. Ainsi, le diamètre de Lₖ ne constitue pas une limitation de débit par rapport au diamètre d'ouverture des vannes de réseau connectées directement à Lₖ.

De préférence, la colonne entière (à l'exception du plateau de tête, exclu par la définition choisie du mot tronçon) est constituée par les tronçons superposés adjacents Sₖ. Dans ce cas, on assimile selon l'invention la ligne de sortie inférieure en fond de colonne à un plateau Pn correspondant au lit d'adsorbant inférieur An. En effet, il n'y a typiquement pas de plateau Pn en dessous du lit d'adsorbant An disposé en fond de colonne, car il n'y a pas de nécessité de distribuer les fluides dans un lit immédiatement inférieur. Aussi, selon l'invention, on considère dans ce cas que le plateau Pn manquant est remplacé par la ligne de sortie inférieure de la colonne, typiquement reliée soit à l'entrée de la même colonne, via une pompe de recirculation, soit à la tête d'une deuxième colonne de séparation.

Comme déja mentionné, le SMB peut fonctionner avec un reflux RE, comprenant de l'extrait, ou typiquement riche en produit recherché obtenu en distillant l'extrait pour éliminer le désorbant (comprenant plus de 50%, ou même 90% voire 99% de produit recherché). De préférence, le dispositif selon l'invention comprend alors un réseau d'alimentation séquentielle RE-Net du reflux RE, ce réseau étant relié à chacun des secteurs Sₖ via une ligne unique de diamètre interne supérieur ou égal à α comprenant une vanne de réseau unique V_{REk}, qui est de diamètre d'ouverture supérieur ou égal à α. Ainsi, le réseau du reflux est raccordé de façon identique à ceux des autres fluides de procédé F, D, R, E.

De façon analogue, le SMB peut aussi fonctionner avec un soutirage séquentiel d'un second raffinat R2, et dans ce cas, le dispositif selon l'invention comprend de préférence un réseau R2-Net relié à chacun des secteurs Sₖ via une ligne unique de diamètre interne supérieur ou égal à α comprenant une vanne de réseau unique V_{REk}, qui est de diamètre d'ouverture supérieur ou égal à α. Ainsi, le réseau du second raffinat est raccordé de façon identique à ceux des autres fluides de procédé F, D, R, E, (RE).

L'invention concerne également un procédé de séparation d'un produit utilisant un dispositif tel que décrit précédemment. Typiquement, au cours d'un cycle:
- on utilise séquentiellement chacune des lignes Lₖ pour la circulation à leur débit nominal de F, D, R, E, et optionnellement un reflux RE et/ou un second raffinat R2 vers ou à partir de chacun des plateaux de Sₖ via en série la vanne de plateau correspondante et la vanne de réseau correspondante,
- on réalise un balayage à débit inférieur à celui des débits nominaux des fluides F, D, R, E, et optionnellement RE et/ou R2, de chacune des lignes de dérivation externes principales Lₖ pendant une partie du temps au moins dans laquelle aucune vanne de réseau reliée à Lₖ n'est ouverte, par un courant interne provenant d'un plateau du dispositif et recyclé vers un autre plateau du dispositif, et l'on stoppe tout balayage interne de Lₖ lorsque qu'une vanne de réseau reliée à Lₖ est ouverte
- on réalise un balayage à débit inférieur à celui des débits nominaux des fluides F, D, R, E de chacune des lignes de dérivation externes secondaires Mₖ pendant une partie du temps au moins, par un courant interne provenant d'un plateau du dispositif et recyclé vers un autre plateau du dispositif.

Le procédé selon l'invention utilise donc le dispositif SMB en réalisant efficacement les balayages par circulation de plateau à plateau via les lignes de dérivation externes Lₖ et Mₖ.

Typiquement, le balayage de Lₖ est réalisé par circulation d'un courant issu du plateau supérieur Pᵢ de Sₖ, recyclé au plateau inférieur Pᵢ₊₁ de Sₖ.

Typiquement également, le balayage de Mₖ est réalisé par circulation d'un courant issu du plateau inférieur Pᵢ₋₁ de Sₖ₋₁, recyclé au plateau supérieur Pᵢ de Sₖ.

Généralement, on réalise un balayage interne de Lₖ à partir du plateau supérieur Pᵢ de Sₖ vers le plateau inférieur Pᵢ₊₁ de Sₖ, dans toute période ou Sₖ n'est pas relié à l'un desdits réseaux fluides d'alimentation séquentielle ou de soutirage séquentiel et qui se trouve immédiatement avant une période ou l'une des vannes de réseaux reliées à Sₖ est ouverte pour l'alimentation ou le soutirage de l'un de ces fluides vers ou à partir du plateau supérieur Pᵢ. Ce balayage interne conduit à l'ouverture de Vᵢ dans la période précédant une période d'alimentation ou de soutirage du plateau Pᵢ (ce qui requiert aussi l'ouverture de Vᵢ) et évite un mouvement d'ouverture ou fermeture de Vᵢ entre ces périodes consécutives. La réduction du nombre de mouvements de vannes réduit l'usure de ces vannes et accroît la fiabilité du dispositif et du procédé associé.

On réalise des balayages internes d'au moins deux et typiquement de la totalité des lignes de dérivation Lₖ. Généralement, pour une ligne Lₖ (ou Mₖ) donnée, le balayage interne dure pendant au moins 20%, souvent au moins 40 %, ou même au moins 50 % du temps.

De préférence, pour chacune des lignes de dérivation Lₖ, on réalise un balayage de Lₖ lors de toute partie du temps dans laquelle aucune vanne de réseau reliée à Lₖ n'est ouverte.

Typiquement, Lₖ est empruntée par chacun des fluides F, D, R, E sur la totalité de sa longueur au cours d'un cycle. Ceci évite toute zone morte dans Lₖ.

Les vannes de plateau Vᵢ₋₁ et Vᵢ des embouts reliés par une ligne de dérivation externe secondaire Mₖ sont de préférence fermées lorsqu'on réalise un balayage de Mₖ. Ceci permet d'éviter un mélange partiel du débit de balayage avec le fluide se trouvant dans Lₖ.

On peut réaliser un balayage de Mₖ lors de toute partie du temps dans laquelle les vannes de plateau Vᵢ₋₁ et Vᵢ des embouts reliés par la ligne de dérivation externe secondaire Mₖ sont fermées.

Selon une variante de réalisation du procédé selon l'invention, on réalise des permutations asynchrones des points d'alimentation et de soutirage des fluides F, D, R, E dans la colonne.

On peut aussi utiliser le dispositif avec des zones chromatographiques dont certaines au moins sont équivalentes à un nombre non entier de lits d'adsorbant, typiquement en Varicol.

L'invention n'est pas liée à une séparation particulière, mais peut être utilisée pour toutes les séparations en lit mobile simulé. On peut par exemple réaliser un procédé de séparation d'un hydrocarbure aromatique, par exemple du paraxylène ou du métaxylène à partir d'une charge d'aromatiques ayant essentiellement 8 atomes de carbone et comprenant cet hydrocarbure.

On peut aussi réaliser un procédé de séparation d'au moins un hydrocarbure normal-paraffinique à partir d'une charge d'hydrocarbures comprenant un tel hydrocarbure, ou un procédé de séparation d'au moins un hydrocarbure oléfinique à partir d'une charge d'hydrocarbures comprenant un tel hydrocarbure.

### Description des figures et fonctionnement des dispositifs représentés:

L'invention sera comprise aisément en suivant la description des figures annexées dans lesquelles:

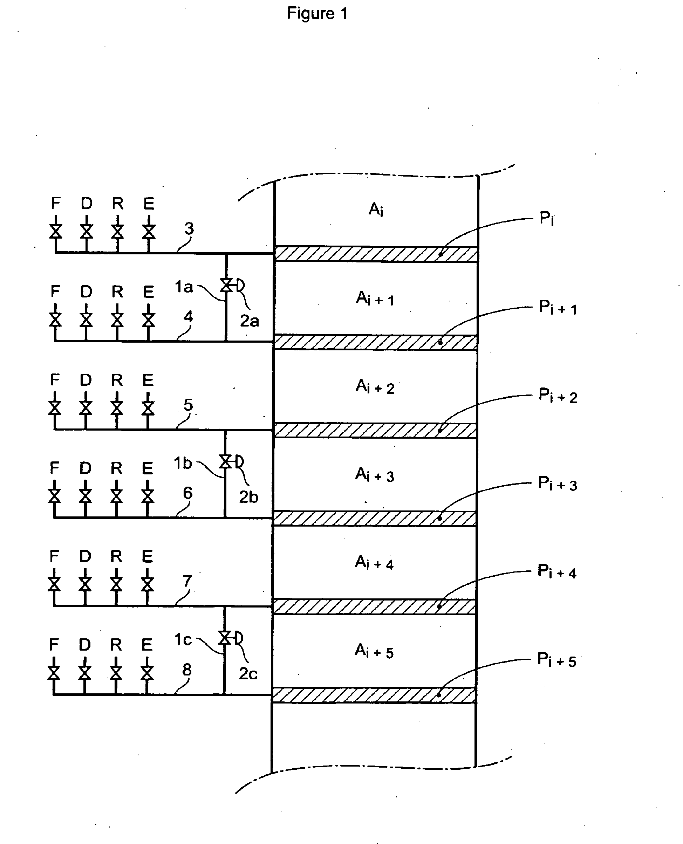

La figure 1 représente schématiquement une partie d'un dispositif SMB selon l'art antérieur, avec les vannes de réseau correspondantes.
La figure 2 représente schématiquement une partie d'un dispositif SMB selon l'invention, comprenant trois tronçons superposés Sₖ, Sₖ₊₁, Sₖ₊₂, avec les dérivations principales, secondaires, les vannes de réseau, les vannes de plateau et les vannes de limitation de débit correspondantes.
La figure 3 représente schématiquement une partie d'un dispositif SMB selon l'invention, comprenant des vannes de limitation de débit situées sur les lignes Lₖ, Lₖ₊₁ elles-mêmes.

On se réfère maintenant à la figure 1 représentant une partie de colonne chromatographique d'un SMB selon l'art antérieur. Chacun des lits d'adsorbants Aᵢ, Aᵢ₊₁, Aᵢ₊₂, Aᵢ₊₃, Aᵢ₊₄, Aᵢ₊₅ est disposé au dessus d'un plateau Pᵢ, Pᵢ₊₁, Pᵢ₊₂, Pᵢ₊₃, Pᵢ₊₄, Pᵢ₊₅ et chacun de ces plateaux est relié par une ligne, respectivement 3, 4, 5, 6, 7, 8 à chacun des 4 réseaux fluides F, D, R, E par une vanne (non référencée). Il y a donc 4 vannes principales par plateau. De plus, les plateaux sont reliés deux à deux par une ligne de dérivation 1a, 1b, 1c, typiquement de relativement petit diamètre, et comprenant une vanne de relativement petit diamètre (inférieur ou égal à une valeur (β), respectivement 2a, 2b, 2c pour permettre le passage d'un débit de dérivation limité : 2 % à 20 % du débit circulant dans la colonne.

Au total, il y a donc, pour chaque plateau Pᵢ, 4 vannes principales de relativement grand diamètre d'ouverture, supérieur ou égal à une valeur α > β (compatible avec les débits nominaux de F, D, R, E, et en moyenne 0,5 vanne de petit diamètre (une pour 2 plateaux), soit en moyenne 4,5 vannes par plateau dont quatre de grand diamètre d'ouverture, supérieur ou égal à α.

Le fonctionnement d'un SMB utilisant une telle colonne est bien connu de l'homme de l'art. Typiquement la vanne 2a, ou 2b, ou 2c d'une ligne de dérivation est ouverte, et régule un débit limité de balayage, lorsque aucun fluide F, D, R, E n'est alimenté ou soutiré de l'un des 2 plateaux reliés par la ligne de dérivation (dérivation temporairement en service). Inversement la vanne 2a, ou 2b, ou 2c d'une ligne de dérivation est fermée lorsque l'un des fluides F, D, R, E est alimenté ou soutiré de l'un des 2 plateaux reliés par la ligne de dérivation (dérivation temporairement hors service).

La figure 2 représente une partie de colonne d'un dispositif selon l'invention comprenant 3 tronçons Sₖ, Sₖ₊₁, Sₖ₊₂, chacun comprenant 2 lits d'adsorbant et 2 plateaux situés immédiatement en dessous. Les 2 plateaux de chaque tronçon sont reliés par une ligne de dérivation principale, de relativement grand diamètre, typiquement supérieur ou égal à α, respectivement Lₖ, Lₖ₊₁, Lₖ₊₂, qui est apte à la circulation des fluides F, D, R, E,... à leur débit nominal. Chaque ligne de dérivation est reliée à un ensemble de 4 vannes de réseau de relativement grand diamètre d'ouverture, supérieur ou égal à α pour l'alimentation et le soutirage séquentiel des fluides de procédé. Contrairement à l'art antérieur, cet ensemble de 4 vannes de réseau alimente non pas 1 mais 2 plateaux.

Ainsi, pour le premier tronçon Sₖ, il y a 4 vannes de réseau V_{Fk}, V_{Dk}, V_{Rk}, V_{Ek} alimentant à la fois Pᵢ et Pᵢ₊₁.

Chaque plateau est par ailleurs relié à la ligne de dérivation correspondante Lₖ, ou Lₖ₊₁, ou Lₖ₊₂ par un embout (correspondant à la partie de ligne horizontale sur la figure) comprenant une vanne de sectionnement commandée à 2 voies unique propre au plateau, appelée vanne de plateau : Vᵢ, Vᵢ₊₁, Vᵢ₊₂, Vᵢ₊₃, Vᵢ₊₄, Vᵢ₊₅. Chaque vanne de plateau inférieure d'un tronçon: Vᵢ₊₁, Vᵢ₊₃, Vᵢ₊₅ possède par ailleurs une petite ligne de dérivation secondaire *Iₖ, Iₖ₊₁, Iₖ₊₂* munie d'une vanne typiquement de relativement petit diamètre: *vᵢ₊₁, vᵢ₊₃, vᵢ₊₅*.

Chaque plateau est également relié à une ligne de dérivation secondaire Mₖ ou Mₖ₊₁ ou Mₖ₊₂ , équipé d'une vanne de relativement petit diamètre V_{Mk} ou V_{Mk+1} ou V_{Mk+2}.

Au total, pour chaque tronçon de 2 plateaux, il y a 4 vannes de réseau de relativement grand diamètre, 2 vannes de plateau, elles aussi de relativement grand diamètre pour permettre la circulation à leur débit nominal de F, D, R, E,... et deux vannes de relativement petit diamètre en dérivation (annexe et secondaire), soit 8 vannes, et donc en moyenne 4 vannes par plateau dont 3 vannes de grand diamètre. On gagne donc une vanne de grand diamètre par plateau si l'on compare ce dispositif avec celui de la figure 1 selon l'art antérieur.

### Le dispositif fonctionne de la façon suivante:

Pour le tronçon Sₖ par exemple, lorsqu'on veut, dans une période donnée, alimenter ou soutirer l'un des fluides F, D, R, E au plateau Pᵢ, on ouvre la vanne de réseau correspondante V_{Fk}, V_{Dk}, V_{Rk}, ou V_{Ek} ainsi que la vanne de plateau Vᵢ. Les autres vannes de réseau du tronçon Sₖ sont alors fermées, ainsi que Vᵢ₊₁, la petite vanne en dérivation secondaire V_{Mk} de la ligne de dérivation secondaire supérieure Mₖ et la petite vanne en dérivation annexe *vᵢ₊₁.* Par contre, la petite vanne de dérivation secondaire V_{Mk+1} de la ligne de dérivation secondaire Mₖ₊₁ est de préférence ouverte

Lorsqu'on veut, dans une autre période, alimenter ou soutirer l'un des fluides F, D, R, E au plateau Pᵢ₊₁, on ouvre la vanne de réseau correspondante V_{Fk} V_{Dk}, V_{Rk}, ou V_{Ek} ainsi que la vanne de plateau Vᵢ₊₁. Les autres vannes de réseau du tronçon Sₖ sont alors fermées, ainsi que Vᵢ. La petite vanne en dérivation annexe *vᵢ₊₁* peut rester fermée. La petite vanne de dérivation secondaire V_{Mk} de la ligne de dérivation secondaire supérieure Mₖ est de préférence ouverte, et la petite vanne de dérivation secondaire V_{Mk+1} de la ligne de dérivation secondaire Mₖ₊₁ est obligatoirement fermée.

Lorsqu'on veut, dans une troisième période, ne pas alimenter ni soutirer l'un des fluides F, D, R, E aux plateaux Pᵢ et Pᵢ₊₁, on ferme les vannes de réseau V_{Fk}, V_{Dk}, V_{Rk}, et V_{Ek}. On réalise alors une circulation d'un débit limité de dérivation dans la ligne Lₖ (soutiré de Pᵢ et injecté dans Pᵢ₊₁) en ouvrant Vᵢ, fermant Vᵢ₊₁ et ouvrant la petite vanne en dérivation annexe *vᵢ₊₁.* On peut ainsi assurer via *lₖ* un petit débit de dérivation. *vᵢ₊₁* est typiquement une vanne de régulation (à ouverture progressive) pilotée par régulation de débit à partir d'un débitmètre non représenté.

Lorsqu'on veut, au cours d'une quatrième période, 1°) ne pas alimenter ni soutirer l'un des fluides F, D, R, E aux plateaux Pᵢ et Pᵢ₊₁, on ferme les vannes de réseau V_{Fk} V_{Dk}, V_{Rk}, et V_{Ek}. Et 2°) avoir un débit de dérivation nul dans la ligne Lₖ, on ferme Vᵢ, Vᵢ₊₁ et la petite vanne en dérivation annexe *vᵢ₊₁.* On réalise alors une circulation d'un débit limité dans la ligne de dérivation secondaire Mₖ et éventuellement dans Mₖ₊₁, sauf lorsque les plateaux Pᵢ₋₁ ou Pᵢ₊₂ sont en phase de'alimentation ou de soutirage, auquel cas la ligne de dérivation secondaire correspondante doit rester hors servive.

Les autres secteurs Sₖ₊₁, Sₖ₊₂ fonctionnent de façon analogue.

Un exemple type de fonctionnement d'un secteur Sₖ est par exemple le suivant, dans lequel les vannes pour le fonctionnement de Sₖ qui sont ouvertes sont mentionnées, les vannes non mentionnées étant fermées: Seules les circulations en dérivation secondaire dans Mₖ, pour le balayage de Pᵢ sont décrites (pas celles dans Mₖ₋₁).

Période 1: Balayage en dérivation de Pᵢ vers Pᵢ₊₁. vannes ouvertes: Vᵢ, *vᵢ₊₁.*

Période 2: Injection de désorbant dans Pᵢ. vannes ouvertes: Vᵢ, V_{Dk}.

Période 3: Injection de désorbant dans Pᵢ₊₁. vannes ouvertes: Vᵢ₊₁, V_{Dk}. Et Balayage en dérivation de Pᵢ₋₁ vers Pᵢ. Vanne ouverte: V_{Mk}.

Période 4: Balayage en dérivation de Pᵢ vers Pᵢ₊₁. vannes ouvertes: Vᵢ, *vᵢ₊₁.*

Période 5: Soutirage de raffinat de Pᵢ. vannes ouvertes: Vᵢ, V_{Rk}.

Période 6: Soutirage de raffinat de Pᵢ₊₁. vannes ouvertes: Vᵢ₊₁, V_{Rk}. Et Balayage en dérivation de Pᵢ₋₁ vers Pᵢ. vanne ouverte: V_{Mk}.

Période 7: Balayage en dérivation de Pᵢ vers Pᵢ₊₁. vannes ouvertes: Vᵢ, *vᵢ₊₁.*

Période 8: Balayage en dérivation de Pᵢ₋₁ vers Pᵢ. vanne ouverte: V_{Mk}.

Période 9: Balayage en dérivation de Pᵢ vers Pᵢ₊₁. vannes ouvertes: Vᵢ, *vᵢ₊₁.*

Période 10: Balayage en dérivation de Pᵢ₋₁ vers Pᵢ. vanne ouverte: V_{Mk}.

Période 11: Balayage en dérivation de Pᵢ vers Pᵢ₊₁. vannes ouvertes: Vᵢ, *vᵢ₊₁.*

Période 12: Injection de charge dans Pᵢ. vannes ouvertes: Vᵢ, V_{Fk}.

Période 13: Injection de charge dans Pᵢ₊₁. vannes ouvertes: Vᵢ₊₁, V_{Fk}. Et Balayage en dérivation de Pᵢ₋₁ vers Pᵢ. vanne ouverte: V_{Mk}.

Période 14: Balayage en dérivation de Pᵢ vers Pᵢ₊₁. vannes ouvertes: Vᵢ, *vᵢ₊₁.*

Période 15: Balayage en dérivation de Pᵢ₋₁ vers Pᵢ. vanne ouverte: V_{Mk}.

Période 16: Balayage en dérivation de Pᵢ vers Pᵢ₊₁. vannes ouvertes: Vᵢ, *vᵢ₊₁.*

Période 17: Balayage en dérivation de Pᵢ₋₁ vers Pᵢ. vanne ouverte: V_{Mk}.

Période 18: Balayage en dérivation de Pᵢ vers Pᵢ₊₁ vannes ouvertes: Vᵢ, *vᵢ₊₁.*

Période 19: Balayage en dérivation de Pᵢ₋₁ vers Pᵢ. vanne ouverte: V_{Mk.}

Période 20: Balayage en dérivation de Pᵢ vers Pᵢ₊₁. vannes ouvertes: Vᵢ, *v*ᵢ₊₁.

Période 21: Soutirage d'extrait de Pᵢ. vannes ouvertes: Vᵢ, V_{Ek.}

Période 22: Soutirage d'extrait de Pᵢ₊₁. vannes ouvertes: Vᵢ₊₁, V_{Ek}. Et balayage en dérivation de Pᵢ₋₁ vers Pᵢ. vanne ouverte: V_{Mk}.

Période 23: Balayage en dérivation de Pᵢ vers Pᵢ₊₁. vannes ouvertes: Vᵢ, *vᵢ₊₁.*

Période 24: Balayage en dérivation de Pᵢ₋₁ vers Pᵢ. vanne ouverte: V_{Mk}.

Les principes permettant d'établir le séquencement préféré sont les suivants :
1) A chaque fois que l'on prélève ou que l'on injecte un des fluides principaux (F, D, R, E) au moyen d'une vanne de réseau dans une ligne de dérivation Lₖ, cette vanne de réseau reste ouverte deux fois de suite (lors de 2 périodes successives). La première fois la vanne de plateau supérieure, ouverte, permet la connection au plateau supérieur Pᵢ, et la vanne de plateau inférieure Vᵢ₊₁ ainsi que la petite vanne *vᵢ₊₁* de contrôle de fluide de dérivation annexe de la ligne *lk* sont fermées. La seconde fois, la vanne de plateau inférieure Vᵢ₊₁, ouverte, permet la connection au plateau inférieur Pᵢ₊₁, et la vanne de plateau supérieure Vᵢ et la petite vanne de contrôle de fluide de dérivation *vᵢ₊₁* sont fermées. De plus la petite vanne de contrôle V_{Mk} de la ligne de dérivation secondaire supérieure Mₖ est ouverte pour mettre en communication les plateaux Pᵢ₋₁ (non représenté) du tronçon Sₖ₋₁ (non représenté) et le plateau Pᵢ du tronçon Sₖ.
2) En dehors des périodes d'injection ou de soutirage des fluides principaux (F, D, R, E), on fait circuler alternativement une fois sur deux un débit de dérivation dans Lₖ. La vanne de plateau supérieure Vᵢ est alors ouverte, la vanne inférieure Vᵢ₊₁ est fermée, et la petite vanne de contrôle *vᵢ₊₁* située en dérivation annexe autour de Vᵢ₊₁ régule le débit de dérivation via la dérivation annexe *lₖ.* Alternativement un débit de dérivation circule dans la ligne de dérivation secondaire supérieure Mₖ régulé par la petite vanne de contrôle V_{Mk}, les deux vannes de plateau Vᵢ₋₁ et Vᵢ étant fermées, pour réaliser une dérivation entre Pᵢ₋₁ et Pᵢ. Ce dernier débit de dérivation ne serait toutefois pas mis en service si le plateau Pᵢ₋₁ était alimenté ou en soutirage par F, D, R, E,...

La figure 3 représente une partie de colonne d'un SMB selon un autre mode de réalisation de l'invention. Les vannes de plateau (non numérotées) ne comprennent pas de petite ligne *Iₖ* de dérivation annexe pour limiter le débit de balayage en dérivation dans Lₖ, comme pour le dispositif de la figure 2. cette fonction est assurée par une vanne typiquement à ouverture progressive : 9_{A} pour Lₖ, et 9_{B} pour Lₖ₊₁. Ceci permet de ne pas utiliser de lignes annexes *Iₖ, Iₖ₊₁,* mais requiert des vannes de relativement grand diamètre 9_{A}, 9_{B} pour ne pas limiter le débit en circulation dans Lₖ.

En alternative, on peut utiliser une vanne de plateau de Lₖ comme vanne de régulation de débit, à la place de la vanne 9_{A} et/ou de la vanne 9_{B}. Cette ou ces vannes doivent alors avoir une étanchéité poussée.

### Meilleur mode de réalisation:

Le meilleur mode de réalisation de l'invention est un SMB dont la ou les colonnes sont essentiellement constituées par des tronçons Sₖ avec des petites vannes *vᵢ₊₁....* en dérivation annexe des plateaux Pᵢ₊₁...., selon la figure 2.

Dans un tel dispositif, il y a 3 vannes de grand diamètre par plateau (6 par secteur Sₖ: V_{Fk}, V_{Dk}, V_{Rk}, V_{Ek}, Vᵢ, Vᵢ₊₁), contre 4 dans l'art antérieur (selon la figure 1). Il y a en moyenne une petite vanne de régulation par plateau (V_{Mk} ou *vᵢ₊₁* pour les 2 plateaux de Sₖ) contre 0,5 dans l'art antérieur, mais cette vanne est beaucoup moins onéreuse, et le nombre total de vannes est diminué (4 contre 4,5).

Le dispositif selon l'invention ainsi décrit peut être utilisé pour un procédé de séparation chromatographique quelconque, notamment pour la séparation d'un hydrocarbure aromatique à partir d'une charge d'aromatiques ayant essentiellement 8 atomes de carbone et comprenant cet hydrocarbure.

En particulier il peut être utilisé pour la séparation de paraxylène à partir d'une coupe aromatique essentiellement composés d'hydrocarbures en C8, en utilisant du toluène ou du paradiéthylbenzène comme désorbant et une zéolithe comme adsorbant comme décrit par exemple dans le brevet FR 2 789 914. Il peut aussi être utilisé pour la séparation de métaxylène à partir d'une coupe aromatique en C8, en utilisant du toluène ou de la tétraline comme désorbant et un adsorbant tel que décrit par exemple dans le brevet US 5,900,523 et les demandes de brevet FR 05/52.485 et FR 05/52.486.

Il peut aussi être utilisé pour la séparation d'une ou de plusieurs normales paraffines (séparées du reste des hydrocarbures) à partir d'un mélange d'hydrocarbures, notamment paraffiniques ou paraffiniques et naphténiques, par exemple en utilisant le normal butane ou le normal pentane comme désorbant (éventuellement l'isooctane comme diluant inerte) et une zéolithe 5A comme adsorbant.

Il peut enfin être utilisé pour la séparation d'au moins une oléfine d'une coupe d'hydrocarbures comprenant un tel hydrocarbure, selon des conditions connues de l'art antérieur, par exemple en utilisant une zéolithe X échangée au calcium.

L'invention n'est pas limitée à la description précédente, et l'homme de l'art pourra utiliser pour sa mise en oeuvre toute autre caractéristique technique connue dans l'état de l'art.

## Revendications

1. Dispositif permettant de séparer au moins un composé à partir d'un mélange comprenant ce composé par adsorption en lit mobile simulé comportant :
au moins une colonne comprenant une pluralité de lits d'adsorbants Aᵢ séparés par des plateaux distributeurs/extracteurs Pᵢ pour l'alimentation et l'extraction séquentielles d'au moins deux fluides d'alimentation : une charge F et un désorbant D, et d'au moins deux fluides de soutirage : un raffinat R et un extrait E, Pᵢ étant disposé entre le lit Aᵢ et le lit immédiatement inférieur Aᵢ₊₁,
le dispositif comprenant également au moins un réseau F-Net de charge, un réseau D-Net de désorbant, un réseau R-Net de raffinat et un réseau E-Net d'extrait, chacun de ces réseaux étant relié à la colonne par une pluralité de lignes comprenant des vannes de sectionnement commandées à 2 voies de diamètre d'ouverture supérieur ou égal à une valeur α, appelées vannes de réseau, pour l'alimentation ou le soutirage séquentiels desdits fluides d'alimentation ou de soutirage,
dans lequel la colonne est divisée, sur la plus grande partie au moins de sa hauteur en une pluralité de tronçons superposés adjacents Sₖ, chaque tronçon Sₖ étant constitué essentiellement par 2 lits d'adsorbant successifs Aᵢ, Aᵢ₊₁ et par les 2 plateaux distributeurs/extracteurs Pᵢ, Pᵢ₊₁ qui sont disposés respectivement immédiatement en dessous de Aᵢ et de Aᵢ₊₁,
chacun des plateaux distributeurs/extracteurs Pᵢ, Pᵢ₊₁ de chacun des secteurs Sₖ est à réseau unique commun pour l'alimentation et le soutirage séquentiels de F, D, R, E ,
les plateaux Pᵢ, Pᵢ₊₁ de chaque tronçon Sₖ sont reliés entre eux par une ligne de dérivation externe principale Lₖ connectée à chacun des plateaux Pᵢ, Pᵢ₊₁ de Sₖ par un embout comprenant une vanne de sectionnement commandée à 2 voies unique propre à ce plateau Pᵢ ou Pᵢ₊₁, appelée vanne de plateau Vᵢ, ou Vᵢ₊₁, de diamètre d'ouverture supérieur ou égal à la valeur α pour l'alimentation ou le soutirage séquentiels desdits fluides d'alimentation ou de soutirage dans ou à partir de Pᵢ,
chacune des dites lignes de dérivation Lₖ comprend au moins un moyen commandé de limitation du débit circulant dans Lₖ, qui est soit installé sur la ligne Lₖ soit en dérivation autour d'une vanne de plateau Vᵢ ou Vᵢ₊₁ d'un plateau de Sₖ,
dans lequel la ligne de dérivation Lₖ de chacun des tronçons Sₖ est reliée à chacun des réseaux F-Net, D-Net, R-Net, E-Net via une ligne unique de diamètre interne supérieur ou égal à α comprenant une vanne de réseau unique, respectivement V_{Fk} ,V_{Dk} ,V_{Rk} ,\/_{Ek} , qui est de diamètre d'ouverture supérieur ou égal à α, pour l'alimentation ou le soutirage séquentiel du fluide correspondant F, D, R ou E vers ou à partir du tronçon Sₖ considéré,
le dispositif comprenant également une pluralité de lignes de dérivation externes secondaires Mₖ, chacune des lignes Mₖ reliant les 2 secteurs adjacents Sₖ₋₁ et Sₖ via 2 points de liaison,
le premier point de liaison étant disposé sur l'embout relié au plateau inférieur Pᵢ₋₁ du secteur supérieur Sₖ₋₁, entre Pᵢ₋₁ et la vanne de plateau Vᵢ₋₁,
le second point de liaison étant disposé sur l'embout relié au plateau supérieur Pᵢ du secteur inférieur Sₖ, entre Pᵢ et la vanne de plateau Vᵢ,
chacune des lignes de dérivation externes secondaires Mₖ comprenant une vanne commandée à 2 voies V_{Mk} de diamètre interne d'ouverture inférieur ou égal à une valeur β, avec β ≤ 0,6 α.

2. Dispositif selon la revendication 1 dans lequel 30 mm ≤ 1,7 × β ≤ α ≤ 600 mm.

3. Dispositif selon l'une des revendications 1 et 2 dans lequel la ligne de dérivation Lₖ a un diamètre intérieur au moins égal au plus grand diamètre d'ouverture des vannes de réseau reliées à Lₖ.

4. Dispositif selon l'une des revendications précédentes dans lequel la colonne entière, à l'exception du plateau de tête, est constituée par lesdits tronçons superposés adjacents Sₖ, la colonne comprenant une ligne de sortie inférieure assimilée à un plateau Pn correspondant au lit d'adsorbant inférieur An.

5. Dispositif selon l'une des revendications précédentes dans lequel chacune des dites lignes de dérivation principales Lₖ comprend au moins un moyen commandé de limitation du débit circulant dans Lₖ, qui est installé en dérivation annexe autour d'une vanne de plateau Vᵢ ou Vᵢ₊₁ d'un plateau de Sₖ.

6. Dispositif selon la revendication 5 dans lequel ledit moyen de limitation du débit circulant dans Lₖ installé en dérivation annexe autour de ladite vanne de plateau Vᵢ ou Vᵢ₊₁ comprend une vanne commandée de diamètre d'ouverture inférieur ou égal à β.

7. Dispositif selon l'une des revendications précédentes dans lequel chacune des dites lignes de dérivation secondaires Mₖ comprend au moins un moyen commandé de limitation du débit circulant dans Mₖ, ce moyen comprenant ladite vanne V_{Mk}.

8. Dispositif selon l'une des revendications précédentes comprenant un réseau d'alimentation séquentielle RE-Net d'un fluide de reflux RE comprenant principalement de l'extrait, ce réseau étant relié à chacun des secteurs Sₖ via une ligne unique de diamètre interne supérieur ou égal à α comprenant une vanne de réseau unique V_{REk}, qui est de diamètre d'ouverture supérieur ou égal à α.

9. Dispositif selon l'une des revendications précédentes comprenant un réseau de soutirage séquentiel R2-Net d'un second raffinat R2, ce réseau étant relié à chacun des secteurs Sₖ via une ligne unique de diamètre interne supérieur ou égal à α comprenant une vanne de réseau unique V_{R2k}, qui est de diamètre d'ouverture supérieur ou égal à α.

10. Procédé de séparation d'un produit utilisant un dispositif selon l'une des revendications précédentes, dans lequel au cours d'un cycle:
- on utilise séquentiellement chacune des lignes Lₖ pour la circulation à leur débit nominal de F, D, R, E, vers ou à partir de chacun des plateaux de Sₖ via en série la vanne de plateau correspondante et la vanne de réseau correspondante,
- on réalise un balayage à débit inférieur à celui des débits nominaux des fluides F, D, R, E de chacune des lignes de dérivation externes principales Lₖ pendant une partie du temps au moins dans laquelle aucune vanne de réseau reliée à Lₖ n'est ouverte, par un courant interne provenant d'un plateau du dispositif et recyclé vers un autre plateau du dispositif, et l'on stoppe tout balayage interne de Lₖ lorsque qu'une vanne de réseau reliée à Lₖ est ouverte
- on réalise un balayage à débit inférieur à celui des débits nominaux des fluides F, D, R, E de chacune des lignes de dérivation externes secondaires Mₖ pendant une partie du temps au moins, par un courant interne provenant d'un plateau du dispositif et recyclé vers un autre plateau du dispositif.

11. Procédé selon la revendication 10 dans lequel le balayage de Lₖ est réalisé par circulation d'un courant issu du plateau supérieur Pᵢ de Sₖ, recyclé au plateau inférieur Pᵢ₊₁ de Sₖ.

12. Procédé selon l'une des revendications 10 et 11 dans lequel le balayage de Mₖ est réalisé par circulation d'un courant issu du plateau inférieur Pᵢ₋₁ de Sₖ₋₁, recyclé au plateau supérieur Pᵢ de Sₖ.

13. Procédé selon l'une des revendications 10 à 12 dans lequel on réalise un balayage interne de Lₖ à partir du plateau supérieur Pᵢ de Sₖ vers le plateau inférieur Pᵢ₊₁ de Sₖ, dans toute période ou Sₖ n'est pas relié à l'un desdits réseaux fluides d'alimentation séquentielle ou de soutirage séquentiel et qui se trouve immédiatement avant une période ou l'une des vannes de réseaux reliées à Sₖ est ouverte pour l'alimentation ou le soutirage de l'un de ces fluides vers ou à partir du plateau supérieur Pᵢ.

14. Procédé selon l'une des revendications 10 à 13 dans lequel pour chacune des lignes de dérivation Lₖ, on réalise un balayage de Lₖ lors de toute partie du temps dans laquelle aucune vanne de réseau reliée à Lₖ n'est ouverte.

15. Procédé selon l'une des revendications 10 à 14 dans lequel les vannes de plateau Vᵢ₋₁ et Vᵢ des embouts reliés par une ligne de dérivation externe secondaire Mₖ sont fermées lorsqu'on réalise un balayage de Mₖ.

16. Procédé selon l'une des revendications 10 à 15 dans lequel on réalise un balayage de Mₖ lors de toute partie du temps dans laquelle les vannes de plateau Vᵢ₋₁, et Vᵢ des embouts reliés par une ligne de dérivation externe secondaire Mₖ sont fermées.

17. Procédé selon l'une des revendications 10 à 16 dans lequel Lₖ est empruntée par chacun des fluides F, D, R, E sur la totalité de sa longueur au cours d'un cycle.

18. Procédé selon l'une des revendications 10 à 17 dans lequel on réalise des permutations asynchrones des points d'alimentation et de soutirage des fluides F, D, R, E dans la colonne.

19. Procédé selon la revendication 18 dans lequel on utilise le dispositif avec des zones chromatographiques dont certaines au moins sont équivalentes à un nombre non entier de lits d'adsorbant.

20. Procédé selon l'une des revendications 10 à 19 de séparation d'un hydrocarbure aromatique à partir d'une charge d'aromatiques ayant essentiellement 8 atomes de carbone et comprenant cet hydrocarbure.

21. Procédé selon la revendication 20 de séparation de paraxylène à partir d'une charge d'hydrocarbures aromatiques ayant essentiellement 8 atomes de carbone.

22. Procédé selon la revendication 20 de séparation de métaxylène à partir d'une charge d'hydrocarbures aromatiques ayant essentiellement 8 atomes de carbone.

23. Procédé selon l'une des revendications 10 à 19 de séparation d'au moins un hydrocarbure normal-paraffnique à partir d'une charge d'hydrocarbures comprenant un tel hydrocarbure.

24. Procédé selon l'une des revendications 10 à 19 de séparation d'au moins un hydrocarbure oléfinique à partir d'une charge d'hydrocarbures comprenant un tel hydrocarbure.

## Claims

1. A device which can separate at least one compound from a mixture comprising said compound by simulated moving bed adsorption, comprising:
at least one column comprising a plurality of adsorbant beds Aᵢ separated by distributor/extractor plates Pᵢ for sequential supply and extraction of at least two supply fluids: a feed F and a desorbant D, and at least two withdrawal fluids: a raffinate R and an extract E, Pᵢ being disposed between the bed Aᵢ and the immediately lower bed Aᵢ₊₁;
the device also comprising at least one feed network F-Net, a desorbant network D-Net, a raffinate network R-Net and an extract network E-Net, each of said networks being connected to the column via a plurality of lines comprising controlled two-way isolating valves with an opening diameter of α or above, termed network valves, for sequential supply or withdrawal of said supply or withdrawal fluids;
in which the column is divided, over at least the major part of its height, into a plurality of adjacent superimposed sections Sₖ, each sector Sₖ being essentially constituted by 2 successive adsorbant beds Aᵢ, Aᵢ₊₁ and by the 2 distributor/extractor plates Pᵢ, Pᵢ₊₁ which are respectively disposed immediately below Aᵢ and Aᵢ₊₁;
each of the distributor/extractor plates Pᵢ, Pᵢ₊₁ of each of the sectors Sₖ has a single common network for sequential supply and withdrawal of F, D, R, E;
plates Pᵢ, Pᵢ₊₁ of each sector Sₖ are connected together via an external principal bypass line Lₖ connected to each of plates Pᵢ, Pᵢ₊₁ of Sₖ via a connector comprising a single two-way controlled isolating valve which belongs to said plate Pᵢ or Pᵢ₊₁, termed a plate valve Vᵢ or Vᵢ₊₁, with an opening diameter which is greater than or equal to the value α for sequential supply or withdrawal of said supply or withdrawal fluids in or from Pᵢ;
each of said bypass lines Lₖ comprises at least one controlled means for limiting the flow moving in Lₖ, which is either installed on the line Lₖ or bypasses a plate valve Vᵢ or Vᵢ₊₁ of a plate of Sₖ;
in which the bypass line Lₖ of each of the sectors Sₖ is connected to each of the networks F-Net, D-Net, R-Net, E-Net via a single line with an internal diameter of α or more comprising a single network valve, respectively V_{Fk}, V_{Dk}, V_{Rk}, V_{Ek}, which has an opening diameter of α or more, for sequential supply or withdrawal of fluid corresponding to F, D, R or E to or from the sector Sₖ under consideration;
the device also comprising a plurality of external secondary bypass lines Mₖ, each of lines Mₖ connecting the 2 adjacent sectors Sₖ₋₁ and Sₖ via 2 connecting points;
the first connecting point being disposed on the connector connecting the lower plate Pᵢ₋₁ of the upper sector Sₖ₋₁ between Pᵢ₋₁ and the plate valve Vᵢ₋₁;
the second connecting point being disposed on the connector connecting the upper plate Pᵢ of the lower sector Sₖ between Pᵢ and the plate valve Vᵢ;
each of the external secondary bypass lines Mₖ comprising a controlled two-way valve V_{Mk} with an internal opening diameter of β or less, in which β ≤ 0.6α.

2. A device according to claim 1, in which 30 mm ≤ 1.7 × β ≤ α ≤ 600 mm.

3. A device according to claim 1 or claim 2, in which the bypass line Lₖ has an internal diameter equal to at least the largest opening diameter of the network valves connected to Lₖ.

4. A device according to one of the preceding claims, in which the whole column with the exception of the head plate is constituted by said adjacent superimposed sections Sₖ, the column comprising a lower outlet line assimilated with a plate Pn corresponding to the lower adsorbant bed An.

5. A device according to one of the preceding claims, in which each of said principal bypass lines Lₖ comprises at least one controlled means for limiting the flow circulating in Lₖ, which is installed in the auxiliary bypass around a plate valve Vᵢ or Vᵢ₊₁ of a plate of Sₖ.

6. A device according to claim 5, in which said means for limiting the flow circulating in Lₖ installed in the auxiliary bypass around said plate valve Vᵢ or Vᵢ₊₁ comprises a controlled valve with an opening diameter of β or less.

7. A device according to one of the preceding claims, in which each of said secondary bypass lines Mₖ comprises at least one controlled means for limiting the flow circulating in Mₖ, said means comprising said valve V_{Mk}.

8. A device according to one of the preceding claims, comprising a sequential supply network RE-Net for a reflux fluid RE principally comprising extract, said network being connected to each of sectors Sₖ via a single line with an internal diameter which is greater than or equal to α, comprising a single network valve V_{REk}, which has an opening diameter of α or more.

9. A device according to one of the preceding claims, comprising a sequential withdrawal network R2-Net for a second raffinate R2, said network being connected to each of the sectors Sₖ via a single line with an internal diameter which is equal to or greater than α, comprising a single network valve V_{R2k} which has a diameter of α or more.

10. A process for separating a product using a device according to one of the preceding claims, in which during a cycle:
• each line Lₖ is used sequentially to circulate F, D, R, E at their nominal flow rate to or from each plate of Sₖ via the corresponding plate valve and the corresponding network valve in series;
• a flush is carried out at a flow rate which is lower than that of the nominal flow rates of fluids F, D, R, E of each of the principal external bypass lines Lₖ during at least part of the time in which no network valve connected to Lₖ is open, using an internal stream deriving from a plate of the device and recycled to another plate of the device, and all internal flushing of Lₖ is stopped when a network valve connected to Lₖ is open;
• a flush is carried out at a flow rate which is lower than that of the nominal flow rates of fluids F, D, R, E of each of the external secondary bypass lines Mₖ for at least part of the time, using an internal stream deriving from a plate of the device and recycled to another plate of the device.

11. A process according to claim 10, in which flushing of Lₖ is carried out by circulating a stream from an upper plate Pᵢ of Sₖ, recycled to the lower plate Pᵢ₊₁ of Sₖ.

12. A process according to claim 10 or claim 11, in which flushing of Mₖ is carried out by circulating a stream from the lower plate Pᵢ₋₁ of Sₖ₋₁, recycled to the upper plate Pᵢ of Sₖ.

13. A process according to one of claims 10 to 12, in which an internal flush of Lₖ is carried out from the upper plate Pᵢ of Sₖ to the lower plate Pᵢ₊₁ of Sₖ, in any period
in which Sₖ is not connected to one of said fluid networks for sequential supply or sequential withdrawal and which is immediately before a period where one of the network valves connected to Sₖ is open to supply or withdraw one of said fluids to or from the upper plate Pᵢ.

14. A process according to one of claims 10 to 13, in which for each of the bypass lines Lₖ, Lₖ is flushed during the entire time in which no network valve connected to Lₖ is open.

15. A process according to one of claims 10 to 14, in which the plate valves Vᵢ₋₁ and Vᵢ of connectors connected via an external secondary bypass line Mₖ are closed when Mₖ is flushed.

16. A process according to one of claims 10 to 15, in which Mₖ is flushed during all of the period when the plate valves Vᵢ₋₁ and Vᵢ of the connectors connected via an external secondary bypass line Mₖ are closed.

17. A process according to one of claims 10 to 16, in which Lₖ is used by each of fluids F, D, R, E over the whole of its length during a cycle.

18. A process according to one of claims 10 to 17, in which asynchronous permutations of the supply and withdrawal points for fluids F, D, R, E are carried out in the column.

19. A process according to claim 18, in which the device is used with chromatographic zones at least some of which are equivalent to a non integral number of beds of adsorbant.

20. A process according to one of claims 10 to 19, for separating an aromatic hydrocarbon from a feed of aromatics essentially containing 8 carbon atoms and comprising said hydrocarbon.

21. A process according to claim 20, for separating para-xylene from a feed of aromatic hydrocarbons essentially containing 8 carbon atoms.

22. A process according to claim 20, for separating meta-xylene from a feed of aromatic hydrocarbons essentially containing 8 carbon atoms.

23. A process according to one of claims 10 to 19, for separating at least one normal-paraffin hydrocarbon from a feed of hydrocarbons comprising said hydrocarbon.

24. A process according to one of claims 10 to 19, for separating at least one olefinic hydrocarbon from a feed of hydrocarbons comprising said hydrocarbon

## Patentansprüche

1. Vorrichtung, die es ermöglicht, mindestens eine Verbindung aus einem Gemisch, das diese Verbindung umfasst, durch Adsorption im simulierten Bewegtbett abzutrennen, umfassend:
mindestens eine Säule, die eine Vielzahl von Adsorptionsbetten Aᵢ umfasst, die durch Einspeisungs-/Extraktionsböden Pᵢ getrennt sind, zur sequentiellen Einspeisung und Extraktion von mindestens zwei Einspeisungsfluiden: einer Beschickung F und einem Desorptionsmittel D, und mindestens zwei Entnahmefluiden: einem Raffinat R und einem Extrakt E, wobei Pᵢ zwischen dem Bett Aᵢ und dem unmittelbar darunter liegenden Bett Aᵢ₊₁ angeordnet ist,
wobei die Vorrichtung mindestens ein Beschickungsnetz F-Net, ein Desorptionsmittelnetz D-Net, ein Raffinatnetz R-Net und ein Extraktnetz E-Net umfasst, wobei jedes dieser Netze mit der Säule durch eine Vielzahl von Leitungen verbunden ist, die gesteuerte 2-Wege-Absperrventile mit einem Öffnungsdurchmesser von größer oder gleich einem Wert α umfassen, die Netzventile genannt werden, zur sequentiellen Einspeisung oder Entnahme der Einspeisungs- oder Entnahmefluide,
wobei die Säule mindestens auf dem größten Teil ihrer Höhe in eine Vielzahl übereinander liegender benachbarter Abschnitte Sₖ gegliedert ist, wobei jeder Abschnitt Sₖ im Wesentlichen aus 2 aufeinander folgenden Adsorptionsbetten Aᵢ, Aᵢ₊₁ und aus den 2 Verteilungs-/Extraktionsböden Pᵢ, Pᵢ₊₁ besteht, die jeweils unmittelbar unter Aᵢ und Aᵢ₊₁ angeordnet sind
wobei jeder der Verteilungs-/Extraktionsböden Pᵢ, Pᵢ₊₁ jeder der Sektoren Sₖ ein einziges gemeinsames Netz zur sequentiellen Einspeisung und Entnahme von F, D, R, E aufweist, wobei die Böden Pᵢ, Pᵢ₊₁ jedes Abschnitts Sₖ untereinander durch eine externe Bypass-Hauptleitung Lₖ verbunden sind, die mit jedem der Böden Pᵢ, Pᵢ₊₁ von Sₖ durch ein Anschlussstück verknüpft ist, das ein einziges gesteuertes 2-Wege-Absperrventil umfasst, das zu diesem Boden Pᵢ oder Pᵢ₊₁ gehört, das Bodenventil Vᵢ oder Vᵢ₊₁ genannt wird, mit einem Öffnungsdurchmesser größer oder gleich dem Wert α zur sequentiellen Einspeisung oder Entnahme der Einspeisungs- oder Entnahmefluide in oder aus Pᵢ,
wobei jede der Bypassleitungen Lₖ mindestens ein Steuerungsmittel zur Begrenzung der Durchflussmenge, die in Lₖ zirkuliert, umfasst, das entweder auf der Leitung Lₖ oder als Ableitung um ein Bodenventil Vᵢ oder Vᵢ₊₁ eines Bodens von Sₖ herum installiert ist
wobei die Bypassleitung Lₖ jedes der Abschnitte Sₖ mit jedem der Netze F-Net, D-Net, R-Net, E-Net über eine einzige Leitung mit einem Innendurchmesser größer oder gleich α verbunden ist, die ein einziges Netzventil V_{Fk}, V_{Dk}, V_{Rk} bzw. V_{Ek} umfasst, das einen Öffnungsdurchmesser größer oder gleich α aufweist, zur sequentiellen Einspeisung oder zur Entnahme des entsprechenden Fluids F, D, R oder E in oder aus dem betrachteten Abschnitt Sₖ,
wobei die Vorrichtung auch eine Vielzahl von externen sekundären Bypassleitungen Mₖ umfasst, wobei jede der Leitungen Mk, die 2 benachbarten Sektoren Sₖ₋₁ und Sₖ über 2 Verbindungspunkte verbindet,
wobei der erste Verbindungspunkt auf dem Anschlussstück, das mit dem unteren Boden Pᵢ₋₁ des oberen Sektors Sₖ₋₁ verbunden ist, zwischen Pᵢ₋₁ und dem Bodenventil Vᵢ₋₁ angeordnet ist,
wobei der zweite Verbindungspunkt auf dem Anschlussstück, das mit dem oberen Boden Pᵢ des unteren Sektors Sₖ verbunden ist, zwischen Pᵢ und dem Bodenventil Vᵢ angeordnet ist,
wobei jede der externen sekundären Bypassleitungen Mₖ, ein gesteuertes 2-Wege-Ventil V_{Mk} mit einem inneren Öffnungsdurchmesser kleiner oder gleich einem Wert β umfasst, wobei gilt β ≤ 0,6 α.

2. Vorrichtung nach Anspruch 1, wobei gilt 30 mm ≤ 1,7 X β ≤ α ≤ 600 mm.

3. Vorrichtung nach einem der Ansprüche 1 und 2, wobei die Bypassleitung Lₖ einen Innendurchmesser von mindestens gleich dem größten Öffnungsdurchmesser der Netzventile aufweist, die mit Lₖ verbunden sind.

4. Vorrichtung nach einem der vorhergehenden Ansprüche, wobei die gesamte Säule mit Ausnahme des Bodens am Kopf aus den übereinander liegenden benachbarten Abschnitten Sₖ besteht, wobei die Säule eine untere Auslassleitung umfasst, die einem Boden Pn gleichgesetzt wird, die dem unteren Adsorptionsbett An entspricht.

5. Vorrichtung nach einem der vorhergehenden Ansprüche, wobei jede der Bypass-Hauptleitungen Lₖ mindestens ein Steuerungsmittel zur Begrenzung der Durchflussmenge, die in Lₖ zirkuliert, umfasst, das in einer zusätzlichen Ableitung um ein Bodenventil Vᵢ oder Vᵢ₊₁ eines Bodens von Sₖ herum installiert ist

6. Vorrichtung nach Anspruch 5, wobei das Mittel zur Begrenzung der Durchflussmenge, die in Lₖ zirkuliert, das in einer zusätzlichen Ableitung um das Bodenventil Vᵢ oder Vᵢ₊₁ herum installiert ist, ein gesteuertes Ventil mit einem Öffnungsdurchmesser kleiner oder gleich β umfasst.

7. Vorrichtung nach einem der vorhergehenden Ansprüche, wobei jede der sekundären Bypassleitungen Mₖ mindestens ein Steuerungsmittel zur Begrenzung der Durchflussmenge, die in Mₖ zirkuliert, umfasst, wobei dieses Mittel das Ventil V_{Mk} umfasst.

8. Vorrichtung nach einem der vorhergehenden Ansprüche, die ein Netz RE-Net zur sequentiellen Einspeisung eines Rückflussfluids RE umfasst, das im Wesentlichen Extrakt umfasst, wobei dieses Netz mit jedem der Sektoren Sₖ über eine einzige Leitung mit einem Innendurchmesser größer oder gleich α verbunden ist, die ein einziges Netzventil V_{REk} umfasst, das einen Öffnungsdurchmesser größer oder gleich α aufweist.

9. Vorrichtung nach einem der vorhergehenden Ansprüche, die ein Netz R2-Net zur sequentiellen Entnahme eines zweiten Raffinats R2 umfasst,
wobei dieses Netz mit jedem der Sektoren Sₖ über eine einzige Leitung mit einem Innendurchmesser größer oder gleich α verbunden ist, die ein einziges Netzventil V_{R2k} umfasst, das einen Öffnungsdurchmesser größer oder gleich α aufweist.

10. Verfahren zur Abtrennung eines Produkts unter Verwendung einer Vorrichtung nach einem der vorhergehenden Ansprüche, wobei im Verlauf eines Zyklus:
- jede der Leitungen Lₖ zur Zirkulation der Fluide F, D, R, E mit ihrer nominalen Durchflussmenge zu oder von jedem der Böden von Sₖ, in Reihe, über das entsprechende Bodenventil und das entsprechende Netzventil sequentiell verwendet wird,
- eine Spülung mit einer geringeren Durchflussmenge als derjenigen der nominalen Durchflussmengen der Fluide F, D, R, E jeder der externen Bypass-Hauptleitungen Lₖ mindestens während eines Zeitabschnitts, in dem kein Netzventil, das mit Lₖ verbunden ist, geöffnet ist, durch einen inneren Strom realisiert wird, der von einem Boden der Vorrichtung stammt und zu einem anderen Boden der Vorrichtung recycelt wird, und jede innere Spülung von Lₖ gestoppt wird, wenn nur ein Netzventil, das mit Lₖ verbunden ist, geöffnet ist,
- eine Spülung mit einer geringeren Durchflussmenge als derjenigen der nominalen Durchflussmengen der Fluide F, D, R, E jeder der externen sekundären Bypassleitungen Mₖ während mindestens eines Zeitabschnitts durch einen inneren Strom realisiert wird, der von einem Boden der Vorrichtung stammt und zu einem anderen Boden der Vorrichtung recycelt wird.

11. Verfahren nach Anspruch 10, wobei die Spülung von Lₖ durch Zirkulation eines Stroms realisiert wird, der aus dem oberen Boden Pᵢ von Sₖ stammt, der zum unteren Boden Pᵢ₊₁ von Sₖ recycelt wird.

12. Verfahren nach einem der Ansprüche 10 und 11, wobei die Spülung von Mₖ durch Zirkulation eines Stroms realisiert wird, der aus dem unteren Boden Pᵢ₋₁ von Sₖ stammt, der zum oberen Boden Pᵢ von Sₖ recycelt wird.

13. Verfahren nach einem der Ansprüche 10 bis 12, wobei eine innere Spülung von Lₖ vom oberen Boden Pᵢ von Sₖ zum unteren Boden Pᵢ₊₁ von Sₖ während jedes Zeitraums realisiert wird, in dem Sₖ nicht mit einem der Fluidnetze zur sequenziellen Einspeisung oder sequentiellen Entnahme verbunden ist, und der sich unmittelbar vor einem Zeitraum befindet, in dem eines der Netzventile, die mit Sₖ verbunden sind, zur Einspeisung oder Entnahme eines dieser Fluide zum oder vom oberen Boden Pᵢ geöffnet ist.

14. Verfahren nach einem der Ansprüche 10 bis 13, wobei für jede der Bypassleitungen Lₖ eine Spülung von Lₖ während jedes Zeitabschnitts realisiert wird, in dem kein Netzventil, das mit Lₖ verbunden ist, geöffnet ist.

15. Verfahren nach einem der Ansprüche 10 bis 14, wobei die Bodenventile Vᵢ₋₁ und Vᵢ der Anschlusstücke, die durch eine externe sekundäre Bypassleitung Mₖ verbunden sind, geschlossen sind, wenn eine Spülung von Mₖ realisiert wird.

16. Verfahren nach einem der Ansprüche 10 bis 15, wobei eine Spülung von Mₖ während jedes Zeitabschnitts realisiert wird, in dem die Bodenventile Vᵢ₋₁ und Vᵢ der Anschlusstücke, die durch eine externe sekundäre Bypassleitung Mₖ verbunden sind, geschlossen sind.

17. Verfahren nach einem der Ansprüche 10 bis 16, wobei Lₖ von jedem der Fluide F, D, R, E auf der Gesamtheit ihrer Länge im Verlauf eines Zyklus benutzt wird .

18. Verfahren nach einem der Ansprüche 10 bis 17, wobei asynchrone Umstellungen der Einspeisungs- und Entnahmepunkte der Fluide F, D, R, E in der Säule realisiert werden.

19. Verfahren nach Anspruch 18, wobei die Vorrichtung mit Chromatographiezonen verwendet wird, von denen mindestens bestimmte mit einer nicht ganzen Zahl der Adsorptionsbetten äquivalent sind.

20. Verfahren nach einem der Ansprüche 10 bis 19 zum Abtrennen eines aromatischen Kohlenwasserstoffs aus einer Beschickung aus Aromaten, die im Wesentlichen 8 Kohlenstoffatome aufweisen und diesen Kohlenwasserstoff umfassen.

21. Verfahren nach Anspruch 20 zum Abtrennen von Para-Xylol aus einer Beschickung aus aromatischen Kohlenwasserstoffen, die im Wesentlichen 8 Kohlenstoffatome aufweisen.

22. Verfahren nach Anspruch 20 zum Abtrennen von Meta-Xylol aus einer Beschickung aus aromatischen Kohlenwasserstoffen, die im Wesentlichen 8 Kohlenstoffatome aufweisen.

23. Verfahren nach einem der Ansprüche 10 bis 19 zum Abtrennen mindestens eines normal-paraffinischen Kohlenwasserstoffs aus einer Beschickung aus Kohlenwasserstoffen, die einen solchen Kohlenwasserstoff umfasst.

24. Verfahren nach einem der Ansprüche 10 bis 19 zum Abtrennen mindestens eines olefinischen Kohlenwasserstoffs aus einer Beschickung aus Kohlenwasserstoffen, die einen solchen Kohlenwasserstoff umfasst.
